# EUROPÄISCHE PATENTANMELDUNG

(11) **EP 4 483 911 A1**
(43) Veröffentlichungstag der Anmeldung: **01.01.2025**
(21) Anmeldenummer: 24166940.7
(22) Anmeldetag: 27.03.2024
(51) Int. Cl.: A61L 2/24, A61L 2/26, B65B 3/00, B67C 3/00

(54) **VORRICHTUNG UND VERFAHREN ZUM BEHANDELN UND INSBESONDERE STERILISIEREN VON BEHÄLTNISSEN**

(30) Priorität: 27.06.2023 DE 102023116964
(71) Anmelder: Krones AG, 93073 Neutraubling (DE)
(72) Erfinder: Effenberger, Harald, 93073 Neutraubling (DE); Feigl, Alexander, 93073 Neutraubling (DE); Fuchs, Florian, 93073 Neutraubling (DE); Hoffmann, Florian, 93073 Neutraubling (DE); Eichenseher, Andreas, 93073 Neutraubling (DE); Kornprobst, Stefan, 93073 Neutraubling (DE); Gette, Viktor, 93073 Neutraubling (DE); Schoenberger, Wolfgang, 93073 Neutraubling (DE); Vornehm, Andreas, 93073 Neutraubling (DE); Senn, Konrad, 93073 Neutraubling (DE); Kammerl, Martin, 93073 Neutraubling (DE); Neubauer, Michael, 93073 Neutraubling (DE); Pense, Andreas, 93073 Neutraubling (DE); Knott, Josef, 93073 Neutraubling (DE); Kiendl, Thomas, 93073 Neutraubling (DE)
(74) Vertreter: Bittner, Bernhard

(57) **Zusammenfassung**

Behältnisbehandlungsvorrichtung (1) zum Transportieren von Kunststoffbehältnissen (10) und insbesondere Kunststoffvorformlingen entlang eines vorgegebenen Transportpfads (T), wobei die Behältnisbehandlungsvorrichtung (1) mehrere Transporteinrichtungen (4, 100, 200, 300) aufweist, die jeweils mindestens ein Halteelement (140, 240) zum Halten eines Kunststoffbehältnisses (10) und Führen des Kunststoffbehältnisses (10) auf einem Abschnitt des Transportpfads (T) aufweisen, wobei eine zweite Transporteinrichtung (100) entlang des Transportpfads (T) unmittelbar auf eine erste Transporteinrichtung (4) folgt und Behältnisse von einer Halteeinrichtung der ersten Transporteinrichtung (4) an eine Halteeinrichtung der zweiten Transporteinrichtung (100) übergebbar sind, wobei der Transportpfad (T), entlang dem die Behältnisse durch die erste Transporteinrichtung (4) führbar sind, zumindest abschnittsweise außerhalb eines Reinraums angeordnet ist und der Transportpfad (T) entlang dem die Behältnisse durch die erste Transporteinrichtung (4) führbar sind, zumindest abschnittsweise von einer Strahlungsabschirmungseinrichtung umgeben ist.

## Beschreibung

Die vorliegende Erfindung betrifft eine Behältnisbehandlungsvorrichtung zum Transportieren von Kunststoffbehältnissen und insbesondere Kunststoffvorformlingen entlang eines vorgegebenen Transportpfads, wobei die Behältnisbehandlungsvorrichtung mehrere Transporteinrichtung aufweist, die jeweils mindestens ein Halteelement zum Halten eines Kunststoffbehältnisses und Führen des Kunststoffbehältnisses auf einem Abschnitt des Transportpfads aufweisen, wobei eine zweite Transporteinrichtung (100) entlang des Transportpfads (T) unmittelbar auf eine erste Transporteinrichtung (4) folgt und Behältnisse von einer Halteeinrichtung der ersten Transporteinrichtung (4) an eine Halteeinrichtung der zweiten Transporteinrichtung (100) übergebbar sind. Weiterhin betrifft die Erfindung ein Verfahren zum Behandeln von Behältnissen, insbesondere zur Sterilisation von Behältnissen.

Aus dem Stand der Technik sind verschiedene Behältnisbehandlungsvorrichtungen bekannt. Die Behandlung kann beispielsweise eine Sterilisation der Behältnisoberflächen umfassen. Oftmals werden in einer solchen Behältnisbehandlungsvorrichtung Vorformlinge behandelt, die in einem folgenden Schritt beispielsweise zu Flaschen oder anderen Gebinden umgeformt werden. Dies hat den Vorteil, dass eine vergleichsweise geringe Fläche behandelt werden muss. Dies hat insbesondere bei Sterilisationsprozessen einen Vorteil, da so eine kleinere Fläche sterilisiert werden muss und Sterilisationsmittel und/oder Energie eingespart werden kann.

Die Sterilisation von Behältnissen erfolgt üblicherweise in einem zumindest weitgehend geschlossenen Gehäuse, um Verunreinigungen von außen zu vermeiden. Ein solches Gehäuse bildet dabei üblicherweise einen sogenannten Reinraum aus.

In einigen Systemen umfasst zumindest ein Sterilisationsprozess eine Beaufschlagung mit Strahlung. Auch eine dafür vorgesehene Strahlenquelle ist üblicherweise zumindest abschnittsweise in einem Gehäuse angeordnet und gibt die Strahlung in das Innere des Gehäuses ab, wobei das Gehäuse abschirmende Eigenschaften für diese Strahlung aufweist. Dadurch können Personen in der Nähe einer solchen Sterilisationseinrichtung vor Streustrahlung geschützt werden. Üblicherweise bildet das Gehäuse mit der abschirmenden Eigenschaft für diese Strahlung nicht nur eine Grenze für die sterilisierende Strahlung sondern auch die Reinraumgrenze.

Insbesondere bei der Sterilisation verschiedener Bereiche von Behältnissen nacheinander ergibt sich die Problematik, dass alle diese Einrichtungen innerhalb des Gehäuses angeordnet sein sollten, um zwischenzeitliche Kontamination der bereits sterilisierten Flächen zu vermeiden. Sind mehrere derartige Sterilisationseinrichtungen innerhalb eines gemeinsamen Gehäuses angeordnet, bilden Sie ein sogenanntes Sterilisationsmodul. Dieses ist aufgrund der vielen Sterilisationseinrichtungen und der Transporteinrichtungen zur Übergabe einzelner Behältnisse von einer Sterilisationseinrichtung zur nächsten oft sehr voluminös und erfordert eine große Stellfläche.

Aus dem internen Stand der Technik der Anmelderin ist es dabei bekannt, Kunststoffvorformlinge nach der Erwärmung in einem Ofen an den Außen- und Innenflächen zu sterilisieren. Durch eine besonders günstige Anordnung von Teilungsverzugssternen, mit denen ein Abstand zweier auf dem Transportpfad direkt aufeinander folgender Behältnisse eingestellt werden kann und der Führung der Behältnisse auf verschiedenen horizontalen Ebenen ist es der Anmelderin gelungen, ein besonders kompaktes Sterilisationsmodul bereitzustellen.

Weiterhin ist bekannt, dass die Sterilisation eines zu befüllenden Behälters neben dem eigentlichen Füllvorgang der zentrale Prozessschritt in einer aseptischen Abfüllanlage ist. Als besonders geeignet hat sich dabei eine ionisierende Strahlung erwiesen, um eine gewünschte Keimreduzierung zu erreichen. Diese Strahlung besteht in den meisten Anwendungen aus beschleunigten Elektronen, die in einer entsprechenden Anlage erzeugt werden und die zu sterilisierenden Behältnisse behandeln, wobei Systeme, die zur Sterilisation eingesetzt werden, meist aus einer Elektronenerzeugungsvorrichtung und einem Strahlfinger zur Entkeimung der inneren Oberflächen sowie einer Elektronenerzeugungsvorrichtung und einem Flächenstrahler zum Entkeimen von äußeren Oberflächen bestehen. Die Behandlungseinrichtungen zur Sterilisation der Außenflächen und die Sterilisation der Innenflächen sind dabei jeweils auf einem Karussell oder Transportstern angeordnet.

Die bei der Sterilisation - beispielsweise durch beschleunigte Elektronen - entstehende Röntgenstrahlung muss dabei durch geeignete Abschirmungen von der Umwelt abgeschirmt werden, so dass die beiden Behandlungseinrichtungen in einer strahlungsabschirmenden Vorrichtung eingebettet bzw. eingehaust sind. Eine derartige Abschirmungseinrichtung ist beispielsweise in EP 2 845 610 A1 näher beschrieben. Bei dieser Abschirmungseinrichtung kann die Strahlungsabschirmung auch an dem Ein- und Auslauf des Sterilisationsmoduls gewährleistet werden, da aufgrund des der Strahlungsbehandlung vorgeschalten Einlaufsterns bzw. des der Strahlungsbehandlung nachgeschalteten Auslaufsterns genügend abschirmende Flächen zwischen der Strahlenquelle und der Ein- und Auslauföffnung angeordnet sind.

Ein solcher Einlaufstern bzw. Auslaufstern ist bei dem aus dem internen Stand der Technik der Anmelderin bekannten kompakten Sterilisationsmodul nicht vorgesehen. Die Sterilisation der Behältnisse findet während des Transports der Behältnisse durch ein Transportstern statt, welcher unmittelbar am Einlauf oder Auslauf in oder aus dem Gehäuse angeordnet ist. Dadurch kann das Gehäuse besonders kompakt ausgebildet sein, die Transfer bzw. Transportstrecke vom Ofen zu der Blasmaschine verkürzt und somit die Verweilzeit der Behältnisse in dem Behandlungsmodul reduziert werden. Jedoch ergibt sich die Problematik, dass (Streu-) Strahlung durch die Ein- und/oder Auslauföffnung aus dem Gehäuse austreten kann.

Es besteht daher der Bedarf, eine Behältnisbehandlungsvorrichtung, insbesondere eine Behältnissterilisationsvorrichtung bereitzustellen, welche die Vorteile der oben beschriebenen kompakten und dennoch einen hohen Durchsatz an Behältnissen bietenden Behältnisbehandlungsvorrichtung bietet, jedoch den Austritt von (Röntgen-) Strahlung effektiv reduziert. Eine solche Behältnisbehandlungsvorrichtung sollte möglichst modular aufgebaut sein und mit anderen vor- oder nachgelagerten Behältnisbehandlungsvorrichtungen wie einer Blasformeinrichtung oder einer Heizeinrichtung kompatibel sein. Weiterhin besteht ein Bedarf an einem Verfahren für eine effektive Behältnisbehandlung auf einem kurzen Transportpfad.

Diese Aufgabe wird durch die Gegenstände der unabhängigen Patentansprüche gelöst. Vorteilhafte Ausführungsformen und Weiterbildungen sind Gegenstand der Unteransprüche.

Eine erfindungsgemäße Lösung des zugrundeliegenden Problems wird somit durch eine Behältnisbehandlungsvorrichtung zum Transportieren von Kunststoffbehältnissen und insbesondere Kunststoffvorformlingen entlang eines vorgegebenen Transportpfads bereitgestellt. Dabei weist die Behältnisbehandlungsvorrichtung mehrere Transporteinrichtungen auf, die jeweils mindestens ein Halteelement zum Halten eines Kunststoffbehältnisses und Führen des Kunststoffbehältnisses auf einem Abschnitt des Transportpfads aufweisen. Eine zweite Transporteinrichtung ist dabei entlang des Transportpfads unmittelbar auf eine erste Transporteinrichtung folgend angeordnet und Behältnisse sind von einer Halteeinrichtung der ersten Transporteinrichtung an eine Halteeinrichtung der zweiten Transporteinrichtung übergebbar.

Wesentlich für eine Lösung des Problems ist, dass der Transportpfad, entlang dem die Behältnisse durch die erste Transporteinrichtung führbar sind, zumindest abschnittsweise außerhalb eines Reinraums angeordnet ist und der Transportpfad, entlang dem die Behältnisse durch die erste Transporteinrichtung führbar sind, zumindest abschnittsweise von einer Strahlungsabschirmungseinrichtung umgeben ist.

Dadurch ist es möglich, dass die Umgebung im Bereich der ersten Transporteinrichtung vor Strahlung der Sterilisationseinrichtung geschützt wird, ohne dass die gesamte erste Transporteinrichtung in einem Reinraum und/oder einem strahlungsschützenden Gehäuse angeordnet sein muss.

Vorzugsweise ist die zweite Transporteinrichtung Teil einer Behältnisoberflächensterilisationseinrichtung, insbesondere einer Behältnisaußenflächensterilisationseinrichtung. Vorzugsweise ist eine Halteeinrichtung der zweiten Transporteinrichtung zumindest zeitweise in den Bereich einer Öffnung einer Gehäusewandung bringbar ist, um ein in den vom Gehäuse umgebenen Raum zu bringendes Behältnis von der ersten Transporteinrichtung aufzunehmen.

Dadurch wird ermöglicht, das Behältnis direkt an eine Behältnisoberflächensterilisationseinrichtung zu übergeben. Dadurch kann auf eine zusätzliche Transporteinrichtung verzichtet werden, welche innerhalb des Gehäuses das eingebrachte Behältnis an die erste entlang des Transportpfads folgende Behältnisoberflächensterilisationseinrichtung übergibt.

Im Folgenden wird eine Behältnisoberflächensterilisationseinrichtung allgemein auch als Behältnisbehandlungseinrichtung bezeichnet. Alle Beispiele, die für eine Sterilisation einer Behältnisoberfläche gegeben sind, sollen dementsprechend auch für jede andere Art einer Behältnisbehandlung als offenbart gelten. Andererseits sollen alle Ausführungsbeispiele, die allgemein für eine Behältnisbehandlungseinrichtung offenbart sind, auch als für die besonders bevorzugten Sterilisation einer Behältnisoberfläche und somit eine Behältnisoberflächensterilisationseinrichtung als offenbart gelten.

Vorzugsweise ist eine Halteeinrichtung der zweiten Transporteinrichtung bezüglich einer Sterilisationseinrichtung, beispielsweise einer Strahlungsquelle relativbeweglich. Ergänzend oder alternativdazu ist in einer besonders bevorzugten Ausführungsform eine Position und/oder Ausrichtung eines Behältnisses im Bereich der Behältnisaußenflächenbehandlungseinrichtung bezüglich einer senkrechten Projektion des Transportpfads veränderbar. Diese Bewegung von Behältnissen relativ zueinander oder bezüglich der Projektion des Transportpfads ermöglicht beispielsweise ein Behältnis gegenüber einer Düse oder Strahlungsquelle zu rotieren und/oder zu kippen, um auch zuvor abgeschattete Oberflächenbereiche für die Behältnisaußenflächenbehandlungseinrichtung zugänglich zu machen.

In einer bevorzugten Ausführungsform weist die erste und/oder die zweite Transporteinrichtung einen rotierbaren Träger auf. Derartige rotierbare Träger sind insbesondere im Bereich der Handhabung von Flaschen oft verwendet, so dass sich bei dieser Ausführungsform eine besonders gute Möglichkeit der Integration der Behältnisbehandlungsvorrichtung in bestehende oder neu auszurüstende Anlagen bietet. Vorzugsweise verlaufen die Rotationsachsen der ersten und/oder der zweiten Transporteinrichtung im Wesentlichen parallel, insbesondere bevorzugt genau parallel. Durch diese Ausgestaltung kann ein besonders ruhiger Lauf der entlang des Transportpfads unmittelbar aufeinander folgenden Träger bezüglich einander erreicht werden.

Bevorzugt weist die die erste und/oder die zweite Transporteinrichtung eine Vielzahl von Halteelementen auf. Dadurch ist der Transport und/oder eine Behandlung mehrerer Behältnisse während einer wiederkehrenden Bewegung der jeweiligen Transporteinrichtung möglich, was den Durchsatz steigert.

In einer bevorzugten Ausführungsform weist die erste und/oder zweite Transporteinrichtung eine Vielzahl von an diesem Träger angeordneten und auf einer Kreisbahn und/oder auf einer Umlaufbahn mit variablem Abstand zum Mittelpunkt führbaren Halteelementen auf. Im Folgenden soll unter "Kreisbahn" auch eine wie oben erwähnte Umlaufbahn um einen Mittelpunkt verstanden werden, bei dem in einem oder mehreren Sektoren geringfügige Abweichungen von der idealen Kreisbahn vorliegen. Diese können beispielsweise wie im Folgenden beschrieben ist dadurch auftreten, dass der Abstand einzelner Haltelemente vom Mittelpunkt individuell verändert wird, um beispielsweise die Kreisbahn in bestimmten Bereichen abzuflachen oder sogar eine weitgehend lineare Führung von Halteelementen und den daran angeordneten Behältnissen in einem Sektor zu ermöglichen. Vorzugsweise weist ein Haltelement in einem ersten Sektor dieser Kreisbahn, in dem eine Aufnahme eines Behältnisses erfolgt, eine Höhe gegenüber einer senkrechten Projektion des Transportpfads auf, welche von derjenigen in einem zweiten Sektor verschieden ist, in welchem eine Abgabe eines Behältnisses erfolgt. Dadurch wird ermöglicht, dass die Übernahme eines Behältnisses (bezüglich der senkrechten Projektion des Transportpfads) auf einer anderen Höhe erfolgen kann als dessen Abgabe. Dies kann vorteilhaft sein, wenn die entlang des Transportpfads benachbarten Transporteinrichtungen auf verschiedenen Höhenniveaus sind.

In einer bevorzugten Ausführungsform wechselt ein Weg, auf dem ein Halteelement der ersten Transporteinrichtung führbar ist, mindestens einmal, vorzugsweise mehrfach den Krümmungsradius. Eine solche Änderung des Krümmungsradius kann einen Wechsel von einem starken Krümmungsradius zu einem geringen Krümmungsradius (mit gleichem Vorzeichen und damit gleicher Krümmungsrichtung) und umgekehrt einschließen. Insbesondere ist jedoch bevorzugt, dass der Krümmungsradius mindestens einmal das Vorzeichen (also die Krümmungsrichtung) ändert. Durch eine Änderung des Krümmungsradius kann erreicht werden, dass die Strahlungsabschirmungseinrichtung einfallende Stählung besonders effektiv vor dem Austreten in die Umgebung hindern kann. Dies kann dadurch erreicht werden, dass in Bereichen mit besonders starkem Krümmungsradius eine besonders effektive Reflexion und/oder Absorption der Strahlung erfolgt. Vorzugsweise bildet die Strahlungsabschirmungseinrichtung durch den (gegebenenfalls mehrmaligen) Wechsel des Krümmungsradius und/oder der Krümmungsrichtung eine Strahlenfalle aus. Vorzugsweise wird die Strahlung in dem Bereich eines starken Krümmungsradius mehrfach reflektiert und/oder absorbiert, so dass die Intensität der Strahlung abgeschwächt wird und/oder die Strahlung wieder in das Gehäuseinnere reflektiert wird.

Vorzugsweise handelt es sich bei der ersten Transporteinrichtung und/oder bei der zweiten Transporteinrichtung um einen Teilungsverzugsstern. Dadurch ist es möglich, dass ein Abstand zwischen zwei auf dem Transportpfad unmittelbar aufeinander folgenden Behältnissen im Bereich der ersten Transporteinrichtung und/oder bei der zweiten Transporteinrichtung veränderbar ist. Dies ermöglicht beispielsweise im Bereich der zweiten Transporteinrichtung, dass das Behältnis in einer individuell einstellbaren Geschwindigkeit an der Behältnisbehandlungseinrichtung, beispielsweise einem Strahlungsemitter vorbeiführbar ist. Somit kann die Behandlung auf die benötigte Dauer angepasst werden, ohne dass die Geschwindigkeit des gesamten Trägers, an welchem die Halteeinrichtung angeordnet ist, angepasst werden muss.

Ebenfalls kann durch die Ausbildung mindestens der ersten Transporteinrichtung und/oder der zweiten Transporteinrichtung als Teilungsverzugsstern gewährleistet werden, dass die Übergabe eines Behältnisses von der ersten Transporteinrichtung auf die zweite Transporteinrichtung verbessert und mit geringen Verlusten läuft, da die Geschwindigkeit des zu übergebenden Behältnisses im Bereich der Übergabe auf eine für die Übergabe besonders geeignete Geschwindigkeit angepasst werden kann. Insbesondere ist eine Synchronisierung der Übergabegeschwindigkeit durch den Teilungsverzugsstern auf die Geschwindigkeit einer Halteeinrichtung der jeweils anderen Transporteinrichtung möglich.

In einer bevorzugten Ausführungsform ist eine Antriebseinrichtung der innerhalb des Gehäuses angeordneten zweiten Transporteinrichtung außerhalb des Gehäuses angeordnet. Insbesondere handelt es sich dabei um eine Antriebseinrichtung, die oberhalb der durch den Transportpfad aufgespannten Fläche angeordnet ist. Dies ermöglicht den Zugang zu der Antriebseinrichtung, beispielsweise zu Wartungsarbeiten, ohne dass dazu das Gehäuse geöffnet werden muss.

Vorzugsweise sind innerhalb des Gehäuses zusätzlich zu der zweiten Transporteinrichtung, welche vorzugsweise einer Behältnisaußenflächenbehandlungseinrichtung zugeordnet ist, höchstens zwei weitere Transporteinrichtungen angeordnet. Dadurch kann das Volumen des vom Gehäuse umschlossenen Raums reduziert sein und die Behältnisbehandlungsvorrichtung besonders kompakt ausgeführt sein. Bei dieser Ausführungsform ist es möglich, die Behältnisse nach der Behandlung durch die Behältnisbehandlungsvorrichtung an eine im Transportpfad folgende Behandlungseinrichtung in derselben Weise abzugeben, wie dies bei den aus dem Stand der Technik bekannten Behandlungsvorrichtungen mit beispielsweise fünf Transporteinrichtungen der Fall ist. Durch die bei dieser Ausführungsform ebenfalls ungerade Anzahl aller Transporteinrichtungen innerhalb des vom Gehäuse umgebenen Raums ist der Drehsinn der entlang des Transportpfads ersten und letzten Transporteinrichtung innerhalb des Gehäuses identisch.

Bevorzugt umschließt das Gehäuse einen Reinraum. Dies ist insbesondere bevorzugt, wenn es sich bei der Behältnisbehandlungsvorrichtung um eine Sterilisationsvorrichtung handelt. So kann innerhalb des Gehäuses zumindest ein Teilbereich steril gehalten werden und eine Kontamination der innerhalb des Gehäuses sterilisierten Behältnisse vermieden werden.

Ergänzend oder alternativ dazu ist vorgesehen, dass der Transportpfad entlang dem die Behältnisse durch die zweite Transporteinrichtung führbar sind, zumindest abschnittsweise innerhalb eines Reinraums angeordnet ist. Dadurch ist eine Oberflächensterilisation während des Transports der Behältnisse mit der zweiten Transporteinrichtung möglich, ohne dass eine Rekontamination der behandelten Oberfläche zu erwarten ist.

Vorzugsweise ist das Gehäuse eine Strahlungsbarriere. Wird zur Sterilisation einer Behältnisoberfläche eine Strahlung eingesetzt, kann so der Austritt dieser Strahlung oder daraus resultierender Streustrahlung aus dem Gehäuse vermieden werden. Dies dient insbesondere dem Schutz von Personen, die sich im Umfeld einer solchen Behältnisbehandlungsvorrichtung aufhalten.

Vorzugsweise schließt sich die Strahlungsabschirmungseinrichtung unmittelbar an eine Öffnung in einem als Strahlungsbarriere ausgebildeten Gehäuse an. Dabei ist insbesondere vorgesehen, dass zwischen dem Gehäuse und der Strahlungsabschirmungseinrichtung keine Strahlung in die Umgebung austreten kann. Insbesondere ist daher bevorzugt, dass die Strahlungsabschirmungseinrichtung sich unmittelbar an Wandungen anschließt, die eine Öffnung in dem Gehäuse umgeben.

Bevorzugt verläuft der Transportpfad, entlang dem die Behältnisse durch die zweite Transporteinrichtung führbar sind, zumindest abschnittsweise in einem Einflussbereich einer Behältnisaußenflächenbehandlungseinrichtung zum Behandeln von Außenflächen der Kunststoffbehältnisse, insbesondere eine Behältnisaußenflächensterilisationseinrichtung und/oder einer Behältnisinnenflächenbehandlungseinrichtung zum Behandeln von Innenflächen der Kunststoffbehältnisse, insbesondere eine Behältnisinnenflächensterilisationseinrichtung.

Vorzugsweise ist eine Halteeinrichtung der zweiten Transporteinrichtung (insbesondere falls die zweite Transporteinrichtung Teil einer Behältnisaußenflächenbehandlungseinrichtung ist) eine das Behältnis innengreifende Halteeinrichtung und/oder (insbesondere falls die zweite Transporteinrichtung Teil einer Behältnisinnenflächenbehandlungseinrichtung ist) eine Halteeinrichtung der Behältnisinnenflächenbehandlungseinrichtung eine das Behältnis außengreifende Halteeinrichtung. Diese Ausgestaltungen ermöglichen es, dass das Behältnis zumindest an einer Behältnisbehandlungseinrichtung, auf einer Seite von einer Halteeinrichtung an einer Behältnisoberfläche gehalten wird, die der zu behandelnden Behältnisoberfläche gegenüber liegt. Damit müssen keine Teile der Halteeinrichtung die zu behandelnde Behältnisoberfläche kontaktieren, so dass diese für die von der Behältnisbehandlungseinrichtung vorzunehmenden Behandlung frei zugänglich ist. Beispielsweise ist es dadurch möglich, die gesamte zu behandelnde Oberfläche mit einem Sterilisationsmedium wie einer Sterilisationsmittellösung oder sterilisierender Strahlung zu beaufschlagen.

Als Behältnis soll im Folgenden jegliches Behältnis verstanden werden, welches zur Aufnahme eines Mediums geeignet ist. Sofern auf ein erstes Behältnis und ein zweites Behältnis Bezug genommen wird, können diese identisch oder verscheiden sein. Identische Behältnisse können jedoch verschiedene Inhalte aufweisen. Handelt es sich bei der Behandlungsvorrichtung beispielsweise um eine Füllvorrichtung, kann ein erstes Behältnis ein gasförmiges Medium enthalten, das zweite Behältnis eine Flüssigkeit oder ein anderes Gas. Auch wäre denkbar, dass es sich bei der Behandlungsvorrichtung um eine Sterilisationsvorrichtung handelt. Ein erstes Behältnis könnte in diesem Fall beispielsweise ein unsteriles Behältnis sein und das zweite Behältnis ein steriles Behältnis.

Bevorzugt handelt es sich bei dem zu behandelnden (ersten und/oder zweiten) Behältnis um Flaschen und/oder Vorformlinge. Die Behandlungsvorrichtung ist bevorzugt eine Sterilisationsvorrichtung beziehungsweise ein Sterilisationsmodul. Denkbar wäre jedoch auch, dass die Behandlungsvorrichtung auch mindestens eine Behandlungseinrichtung aufweist, die ausgewählt ist aus einer Gruppe, die Verschlusseinrichtung, Blasstation, Befülleinrichtung, Heizeinrichtung, Kühleinrichtung und Etikettiereinrichtung umfasst.

Vorzugsweise erstreckt sich die Strahlungsabschirmungseinrichtung ausgehend von einem Übergabepunkt oder Übergabebereich, an welchem Behältnisse von einer Halteeinrichtung der ersten Transporteinrichtung an eine Halteeinrichtung der zweiten Transporteinrichtung übergebbar sind, abschnittsweise entlang des Transportpfads, auf welchem die Behältnisse durch die erste Transporteinrichtung führbar sind. Somit ist auch ein Abschnitt des Transportpfads von der Strahlungsabschirmungseinrichtung umgeben, welcher nicht zwingend im Reinraum liegt.

Vorzugsweise erstreckt sich die Strahlungsabschirmungseinrichtung auch entlang einer Richtung, in welcher die Halteeinrichtung der ersten Transporteinrichtung nach der Übergabe eines Behältnisses an eine Halteeinrichtung der zweiten Transporteinrichtung bewegbar ist. Dadurch kann eine besonders gute Abschirmung gegen den Austritt von Strahlung erreicht werden. Die Abschirmung ist bei dieser Ausgestaltung beidseits des Übergabebereichs der Behältnisse auf dem Pfad gegeben, welcher von dem Halteelement zurückgelegt wird.

Insbesondere bevorzugt erstreckt sich die Strahlungsabschirmungseinrichtung entlang eines Kreisbogens, auf welchem die Halteeinrichtung der ersten Transporteinrichtung führbar ist. Bei dieser einem Kreisbogen folgenden Ausführung der Strahlungsabschirmungseinrichtung kann diese besonders kompakt ausgeführt sein.

Vorzugsweise umschließt die Strahlungsabschirmungseinrichtung einen Pfad, auf dem eine Halteeinrichtung der ersten Transporteinrichtung bewegbar ist, zumindest abschnittsweise. Dabei ist in diesem Abschnitt der ersten Transporteinrichtung insbesondere ein Querschnitt senkrecht zur Transportrichtung vollständig von der Strahlungsabschirmungseinrichtung umgeben. Dadurch ist eine besonders gute Abschirmung und damit Sicherheit für die Umgebung gegeben. Ergänzend oder alternativ dazu weist die Strahlungsabschirmungseinrichtung einen Querschnitt auf, welcher im Wesentlichen die Form des Buchstaben Omega (S2) hat.

Bevorzugt ist die Strahlungsabschirmungseinrichtung mindestens zweiteilig ausgebildet. In einer bevorzugten Ausführungsform sind die mindestens zwei Strahlungsabschirmungseinrichtungsteile voneinander trennbar sind. Dadurch ist es möglich, die Teile der Strahlungsabschirmungseinrichtung voneinander zu trennen, beispielsweise um einen Innenraum der Strahlungsabschirmungseinrichtung für Wartungsarbeiten zugänglich zu machen. Insbesondere ist bevorzugt, dass die Strahlungsabschirmungseinrichtungsteile durch einen Antrieb voneinander beabstandbar sind.

Ergänzend oder alternativ dazu weist die Strahlungsabschirmungseinrichtung in einem ersten Strahlungsabschirmungsbereich, welcher entlang des Transportpfads stromaufwärts eines Übergabepunkts oder Übergabebereichs, an welchem Behältnisse von einer Halteeinrichtung der ersten Transporteinrichtung an eine Halteeinrichtung der zweiten Transporteinrichtung übergebbar sind, angeordnet ist (im Folgenden auch als vorangehender Strahlungsabschirmungsbereich bezeichnet), in zumindest einer senkrecht zum Transportpfad angeordneten Richtung einen größeren Querschnitt auf als in einem zweiten Strahlungsabschirmungsbereich, in welchem die Halteeinrichtung der ersten Transporteinrichtung nach der Übergabe eines Behältnisses an eine Halteeinrichtung der zweiten Transporteinrichtung bewegbar ist (im Folgenden auch als nachfolgender Strahlungsabschirmungsbereich bezeichnet). Diese Ausführungsform ist besonders vorteilhaft, da die Halteeinrichtung der ersten Transporteinrichtung stromabwärts des Übergabepunkts oder Übergabebereichs nicht mehr mit einem Behältnis bestückt ist und daher weniger Raum beansprucht als stromaufwärts des Übergabepunkts oder Übergabebereichs. Dadurch kann der Querschnitt senkrecht zum Transportpfad im nachfolgenden Übergabebereich geringer gewählt werden. Dies wiederum führt dazu, dass bei geringerer Länge des nachfolgenden Strahlungsabschirmungsbereichs ähnlich viele Reflexionen und Absorptionen der Strahlung erfolgen können, wie im vorangehenden Strahlungsabschirmungsbereich mit größerem Querschnitt. Dementsprechend kann eine dem vorangehenden Strahlungsabschirmungsbereich ähnliche Abschirmleistung des nachfolgenden Strahlungsabschirmungsbereichs bereits bei geringerer Länge erreicht werden.

Vorzugsweise weist der nachfolgende Strahlungsabschirmungsbereich zumindest abschnittsweise einen sich ausgehend von dem Übergabepunkt oder Übergabebereich ausgehend verbreiternden Querschnitt auf. Dadurch kann vermieden werden, dass eventuell nicht korrekt an die zweite Transporteinrichtung übergebene Behältnisse sich in dem nachfolgenden Strahlungsabschirmungsbereich verkeilen und ein Haltelement beschädigen.

Alternativ oder ergänzend dazu kann es vorteilhaft sein, im nachfolgenden Strahlungsabschirmungsbereich Bereiche zum Auswerfen und/oder Ausschleusen von (nicht korrekt an die zweite Transporteinrichtung übergebenen Behältnissen vorzusehen.

Vorzugsweise ist die Strahlungsabschirmungseinrichtung zumindest abschnittsweise nicht eine Reinraumgrenze. In dieser Ausführungsform sind Reinraumgrenze und Strahlungsabschirmung somit zumindest abschnittsweise, vorzugsweise im Bereich der Strahlungsabschirmungseinrichtung, voneinander getrennt. Insbesondere ist bevorzugt, dass die Strahlungsabschirmungseinrichtung zumindest in einem Bereich keine Reinraumgrenze bildet, in welchem sich die Strahlungsabschirmungseinrichtung entlang eines Pfads erstreckt, auf dem eine Halteeinrichtung der ersten Transporteinrichtung bewegbar ist. Die erste Transporteinrichtung kann somit unsteril ausgeführt sein. Dies vereinfacht die Zugänglichkeit und die Wartung der ersten Transporteinrichtung.

Insbesondere bietet dies jedoch dann einen Vorteil, wenn die Behandlung durch die Behältnisaußenflächenbehandlungseinrichtung und/oder die Behältnisinnenflächenbehandlungseinrichtung eine Höhenverschiebung des Behältnisses vorsieht. In diesem Fall ist es nämlich nicht notwendig, dass das Behältnis mehrfach in der Höhe verschoben wird, beispielsweise hoch und wieder runter in die Ausgangsposition. Vielmehr ist denkbar, dass während der Behandlung die Höhenverschiebung lediglich über die für den Prozess unbedingt notwendige Strecke erfolgt und die Halteeinrichtung erst nach Abschluss des Prozesses und der Abgabe des behandelten Behältnisses wieder auf diejenige Höhe verschoben wird, in der ein weiteres Behältnis aufgenommen wird. Dadurch ist es insbesondere bei rotierenden Behältnisflächenbehandlungseinrichtungen möglich, dieses Verschieben des nicht von einem Behältnis besetzten Halteelements in einem Sektor einer Kreisbahn vorzunehmen, in welchem keine Behandlung eines Behältnisses vorgenommen wird und welcher somit ungenutzt bleiben würde.

Bevorzugt ist eine Behältnisaufnahme beziehungsweise eine Halteeinrichtung ein passives Element, beispielsweise eine Klammer. Aktive Einrichtungen, die zur Übernahme und Übergabe notwendig sind, beispielsweise zum Eindrücken eines Vorformlings in eine Klammer und das Herausziehen aus einer Klammer entgegen der Haltekraft der Klammer, sind bevorzugt auf die Übergabeeinrichtung und die Übernahmeeinrichtung ausgelagert. In einer bevorzugten Ausführungsform ist eine Halteeinrichtung der Behältnisaußenflächenbehandlungseinrichtung und/oder der Behältnisinnenflächenbehandlungseinrichtung ein aktives Element. Bei einem solchen aktiven Element ist vorzugsweise die Kraft, mit der ein Behältnis gegriffen wird, veränderbar. Beispielsweise kann diese Veränderung sektorabhängig erfolgen. Diese Ausführungsform ist bevorzugt, da mit den aktiven Haltelementen / Klammern eine bessere und sicherere Übernahme und/oder Übergabe gewährleistet werden kann.

Weiterhin wird die der Erfindung zugrundeliegende Aufgabe durch ein Verfahren zum Transportieren von Kunststoffbehältnissen und insbesondere Kunststoffvorformlingen entlang eines vorgegebenen Transportpfads gelöst. Dabei werden die Kunststoffbehältnisse durch mehrere Transporteinrichtungen transportiert, wobei jeweils mindestens ein Halteelement ein Kunststoffbehältnis hält und dieses Kunststoffbehältnis auf einem Abschnitt des Transportpfads führt. Ein Kunststoffbehältnis wird von einer Halteeinrichtung einer ersten Transporteinrichtung an eine Halteeinrichtung einer zweiten Transporteinrichtung übergeben. Insbesondere zeichnet sich das Verfahren dadurch aus, dass die Behältnisse durch die erste Transporteinrichtung auf einem Transportpfad geführt werden, der zumindest abschnittsweise außerhalb eines Reinraums angeordnet ist und die Behältnisse durch die erste Transporteinrichtung auf einem Transportpfad geführt werden, der zumindest abschnittsweise von einer Strahlungsabschirmungseinrichtung umgeben ist.

In einer ersten Variante dieses Verfahrens wird die Lösung der oben genannten Aufgabe dadurch erreicht, dass eine Halteeinrichtung der zweiten Transporteinrichtung zumindest zeitweise in den Bereich einer Öffnung einer Gehäusewandung gebracht wird, um ein in den vom Gehäuse umgebenen Raum zu bringendes Behältnis aufzunehmen. Bei der Übergabe durch diese Öffnung ist das Behältnis vorzugweise zumindest abschnittsweise von der Strahlungsabschirmungseinrichtung umgeben. Wie oben bereits bezüglich der Vorrichtung beschrieben wurde, bietet dies die Möglichkeit, das vom Gehäuse umschlossene Volumen gering zu halten und gleichzeitig eine effiziente Abschirmung der Umgebung gegenüber der im Gehäuse auftretenden Strahlung zu gewährleisten.

Diese und alle weiteren dargestellten Lösungen können ergänzend oder alternativ zu der oben beschriebenen ersten Lösung verwirklicht sein. Auch die im Rahmen bevorzugter Varianten beschriebenen Verfahrensschritte und Ausführungsformen der beschriebenen Vorrichtung sind nicht auf diejenige Lösung beschränkt, in deren Zusammenhang sie erstmals erwähnt sind. Vielmehr können bevorzugte Verfahrensschritte und Ausführungsformen auch Vorteile für Verfahren und/oder Vorrichtungen bringen, die nicht im unmittelbaren Zusammenhang mit dieser Variante beschrieben wurden, sofern diese Kombination technisch realisierbar ist.

Bevorzugt erstreckt sich eine Längsachse eines Behältnisses während dessen Transports entlang des Transportpfads im Wesentlichen Senkrecht zu einer durch den Transportpfad aufgespannten Ebene. Das Behältnis erstreckt sich somit in einer Höhenrichtung, die senkrecht zum Transportpfad beziehungsweise der durch den Transportpfad aufgespannten Ebene steht. Vorzugsweise ist die Senkrechte der senkrechten Projektion des Transportpfads parallel zu der Höhe eines, bevorzugt jedes Behältnisses. Die Höhenrichtung über der senkrechten Projektion des Transportpfads ist somit parallel zu der Höhenrichtung des oder der Behältnisse.

Vorzugsweise ist die Längsachse eines Behältnisses während dessen Transports entlang des Transportpfads im Wesentlichen parallel zu einer Rotationsachse mindestens eines Trägers der ersten und/oder zweiten Transporteinrichtung.

Vorzugsweise weist wenigstens eine erste und/oder eine zweite Transporteinrichtung eine Vielzahl von Halteelementen, insbesondere Halteklammern und/oder Dorne bzw. Haltedorne, zum Halten der Kunststoffbehältnisse während des Transports auf. Dadurch wird der gleichzeitige Transport einer Vielzahl von Behältnissen ermöglicht.

Wie oben dargelegt, handelt es sich in einer bevorzugten Ausführungsform bei der ersten Transporteinrichtung um einen (ersten) Teilungsverzugsstern und bei der zweiten Transporteinrichtung um einen (zweiten) Teilungsverzugsstern. Ein Teilungsverzugsstern ist dabei dazu geeignet und bestimmt die Teilung aufeinanderfolgender bzw. benachbart transportierter Kunststoffbehältnisse zu verändern und insbesondere zu vergrößern und/oder zu verringern. Bevorzugt wird dies dadurch erreicht, dass die Halteelemente der ersten Transporteinrichtung und die Halteelemente der zweiten Transporteinrichtung beweglich und/oder schwenkbar an der jeweiligen Transporteinrichtung / dem jeweiligen Teilungsverzugsstern gelagert sind und insbesondere radial oder tangential gegenüber dem Transportpfad der jeweiligen Transporteinrichtung schwenkbar gelagert sind.

Teilungsverzugssterne werden demnach immer dort eingesetzt, wo Teilungen variiert bzw. verändert werden sollen. Sind noch andere Funktionen nötig, werden diese in der Regel nicht auf dem Teilungsverzugsstern ausgeführt, sondern es sind weitere Transportsterne oder Transporteinrichtungen vor und/oder nach den Teilungsverzugssternen notwendig. Beispielsweise sind bei einer aus dem internen Stand der Technik der Anmelderin bekannten Vorrichtung zwei Transportsterne notwendig, um die Behältnisse mit Haltedornen für eine Außenbehandlung aufzunehmen und nachgelagert ein Teilungsverzugsstern, welcher nur dazu vorgehsehen ist eine Teilung zu ändern. Demnach können aus dem Stand der Technik bekannte Teilungsverzugssterne nur Bewegungen horizontal, d.h. radial und/oder tangential zu einer Ebene, genauer eines Umfangs, des (Teilungsverzugs)Sterns oder bezogen auf eine Längsrichtung des Kunststoffbehältnisses, ausführen, nicht jedoch in anderen Ebenen wie vertikal, d.h. senkrecht zu einer Ebene des (Teilungsverzugs)Sterns bezogen auf den Umfang des (Teilungsverzugs-) Sterns oder bezogen auf die Längsrichtung des Kunststoffbehältnisses. Diese Bewegungen werden stattdessen üblicherweise an anderen bzw. den zusätzlichen Transporteinheiten durchgeführt.

Bevorzugt ist der Behältnisbehandlungsvorrichtung eine Heizvorrichtung vorgeschaltet, welche die Kunststoffbehältnisse und insbesondere Kunststoffvorformlinge auf eine vorgegebene Temperatur erwärmt. Die erste Transporteinrichtung ist vorteilhaft stromabwärts der Heizvorrichtung angeordnet, so dass eine Übergabe der Kunststoffbehältnisse von der Heizvorrichtung an die Behältnisbehandlungsvorrichtung, d.h. von einem Auslaufstern (erste Transporteinrichtung) der Heizvorrichtung an einen Einlaufstern (zweite Transporteinrichtung) der Behältnisbehandlungsvorrichtung mit zwei Teilungsverzugssternen durchgeführt wird, ohne dass zwischen diesen Teilungsverzugssternen weitere Transportsterne oder Transporteinrichtungen angeordnet sind.

Das oben genannte Gehäuse weist bevorzugt eine Einhausung auf, innerhalb welcher bevorzugt ein Reinraum ausgebildet wird. Die erste Transporteinrichtung ist dabei bevorzugt außerhalb des Gehäuses und damit außerhalb des Reinraums angeordnet und die zweite Transporteinrichtung ist bevorzugt innerhalb des Gehäuses und insbesondere innerhalb des Reinraums angeordnet. Die erste Transporteinrichtung übergibt die Kunststoffbehältnisse demnach bevorzugt an die zweite Transporteinrichtung, welche innerhalb eines die Umgebung abschirmenden Gehäuses angeordnet ist. Die zweite Transporteinrichtung bzw. der zweite Teilungsverzugsstern ist demnach bevorzugt aseptisch ausgebildet.

Die erste Transporteinrichtung bzw. der stromabwärts der Heizvorrichtung angeordnete Teilungsverzugsstern übergibt die Kunststoffbehältnisse demnach bevorzugt direkt an einen weiteren Teilungsverzugsstern, an welchem auch direkt eine Außenbehandlung von Kunststoffbehältnissen vorgenommen wird. Im Stand der Technik ist dabei üblicherweise nach dem ersten Teilungsverzugsstern innerhalb des Gehäuses zuerst ein Einlaufstern vorgesehen, welcher die Behältnisse an den nachfolgenden Transportstern für die Außenentkeimung übergibt, welcher die Behältnisse wiederum nach der Außenbehandlung an den zweiten Teilungsverzugsstern übergibt.

Durch die erfindungsgemäße Behältnisbehandlungsvorrichtung bzw. die vorgeschlagene Übergabe von einem Teilungsverzugsstern an direkt einen nachfolgenden Teilungsverzugsstern, an welchen auch die Außenbehandlung vorgenommen wird, entfallen demnach der in dem Gehäuse angeordnete Einlaufstern und der Behandlungs-Transportstern, so dass das Gehäuse insbesondere auch insgesamt kleiner ausgeführt werden kann. Die notwendige Strahlungsabschirmung kann durch die vorzugsweise von der Reinraumgrenze getrennte und im Bereich der ersten Transporteinrichtung angeordnete Strahlungsabschirmungseinrichtung gewährleitet werden.

Die Übergabe eines Behältnisses von der ersten an die zweite Transporteinrichtung erfolgt vorzugsweise im Bereich eines Übergabefensters, welches in einem Gehäuse, welches auch eine Reinraumgrenze bildet, angeordnet ist. Im Bereich dieses Übergabefensters ist die Teilung der Kunststoffbehältnisse bevorzugt variabel, so dass der Teilungsverzug des ersten Teilungsverzugssterns optimal auf die Anforderungen des zweiten Teilungsverzugssterns ausgelegt ist bzw. an diesen anpassbar ist.

Bei einer bevorzugten Ausführungsform weist das Übergabefenster eine Breite auf, die zwischen 250 mm und 320 mm, bevorzugt zwischen 270 mm und 310 mm und besonders bevorzugt zwischen 285 mm und 300 mm liegt. Vorteilhaft ist das Übergabefenster möglichst klein ausgeführt, so dass eine eventuelle Sterilität im Inneren des Gehäuses gewahrt werden kann und gleichzeitigt das Austrittsfenster für mögliche aus dem Gehäuse austretenden Strahlung verringert wird. Bevorzugt ist das Übergabefenster variabel ausgestaltet, so dass dessen Breite veränderbar ist. Dies ist beispielsweise vorteilhaft, da das Übergabefenster so an verschiedene Vorformlingstypen und -größen anpassbar ist.

Bei einer weiteren bevorzugten Ausführungsform weist die zweite Transporteinrichtung eine Hub- und Dreheinrichtung auf, welche eine Bewegung des Halteelements der zweiten Transporteinrichtung in vertikaler und/oder horizontaler Richtung bezüglich einer Längsachse des Kunststoffbehältnisses und eine Drehbewegung des Kunststoffbehältnisses und/oder des Halteelements entlang der Längsachse ermöglicht. Bevorzugt ist jedem Halteelement der zweiten Transporteinrichtung eine eigene Hub- und Dreheinrichtung zugeordnet, so dass die Kunststoffbehältnisse unabhängig voneinander gedreht werden können und/oder die Halteelemente unabhängig voneinander bewegt werden können. Jeder Hub- und Dreheinrichtung ist insbesondere zur Ausführung der Drehbewegung des Kunststoffbehältnisses bevorzugt eine Antriebseinrichtung zugeordnet, welche in bekannter Weise ausgeführt ist. Die Hubbewegung des Halteelements in vertikaler Richtung wird bevorzugt durch eine oder mehrere Hubkurven und wenigstens eine Führungskurve realisiert.

Um die Übergabe von der ersten Transporteinrichtung an die zweite Transporteinrichtung und insbesondere von dem ersten Teilungsverzugsstern and den zweiten Teilungsverzugsstern zu ermöglichen ist demnach zusätzlich eine Hub- und Dreheinrichtung an der zweiten Transporteinrichtung / dem zweiten Teilungsverzugsstern angeordnet, welche bevorzugt zur Aufnahme der Kunststoffbehältnisse geeignet und bestimmt ist.

Die Hub- und Dreheinrichtung wird dabei benötigt, um einerseits die Kunststoffbehältnisse aufzunehmen und innen halten zu können, wobei bevorzugt die Übergabe von den Halteelementen und insbesondere Halteklammern der ersten Transporteinrichtung auf die Halteelemente und insbesondere Haltedorne der zweiten Transporteinrichtung durch einen Bewegung der Halteelemente der zweiten Transporteinrichtung auf die Behältnisse zu und demnach bevorzugt in vertikaler Richtung bzw. in der Längsrichtung der Behältnisse erfolgt, so dass das Halteelement in Richtung des Behältnisses bewegt wird. Andererseits dient die Hub- und Dreheinrichtung dazu, die Behältnisse vor der Behältnisaußenflächenbehandlungseinrichtung und insbesondere Behältnisaußenflächensterilisationseinrichtung, um deren Längsachse zu rotieren, so dass eine am Umfang gleichmäßig verteilte Strahlungsleistung und dadurch resultierende gleichmäßige Entkeimungsleistung auf die Behältnisse aufgebracht werden kann.

Die Halteelemente der ersten Transporteinrichtung sind dabei bevorzugt Halteklammern, welche die Kunststoffbehältnisse an einer Außenwandung und insbesondere unterhalb oder oberhalb des Tragrings der Behältnisse halten. Die Haltelemente der zweiten Transporteinrichtung sind bevorzugt Haltedorne, welche in die Behältnisse eingeführt werden und diese an einer Innenwandung halten. Die Kunststoffbehältnisse werden demnach bevorzugt von einem Außen-Halteelement an ein Innen-Halteelement und insbesondere einer Außen-Halteklammer an einen Innen-Haltedorn übergeben.

Bevorzugt ist ein Abschnitt des Gehäuses und insbesondere ein oberer Abschnitt des Gehäuses stationär angeordnet und ein weiterer Abschnitt, insbesondere ein unterer Abschnitt, bei welchem es sich um einen Boden handelt, beweglich und insbesondere absenkbar angeordnet. Bei dem unteren Abschnitt handelt es sich bevorzugt - wie auch beim oberen Abschnitt - um eine Abschirmungseinrichtung, wie insbesondere eine Strahlenschutzwand. Die Anordnung der beiden Abschirmungseinrichtungen ist dabei derart gestaltet, so dass sich die Hub- und Dreheinrichtung und insbesondere wenigstens eine Führungsrolle der Hub- und Dreheinrichtung durch einen Spalt zwischen den beiden Abschirmungseinrichtungen hindurch auf einer Laufbahn bewegen kann.

Die Strahlenschutzwände des Gehäuses und/oder die Strahlungsabschirmungseinrichtung sind vorteilhaft aus einem besonderen strahlungsabschirmenden Material gefertigt zum Beispiel aus mit Edelstahl eingehaustem Blei oder aus Wolfram bzw. aus einem Wolfram-Sinter Verbund oder mit einem Material mit ähnlichen Eigenschaften.

Gleichwohl sind bevorzugt Teile der Strahlungsabschirmungseinrichtung auch mit Edelstahl oder mit Gussmaterial ausgeführt. Das Anpassen des abschirmenden Materials an eine bessere Strahlendichtigkeit bringt vor allem eine wesentlich geringere Wandstärke der Abschirmung und somit die Möglichkeit, sich mit der Hub-Dreheinheit zwischen den beiden Schutzwänden hindurch auf der Laufbahn des Teilungsverzugssterns zu bewegen bzw. die Abschirmung in die Hub-Drehvorrichtung ragen zu lassen, ohne sie über Gebühr verbreitern bzw. vergrößern zu müssen.

Die Form der Strahlungsabschirmungseinrichtung folgt bevorzugt der Laufbahn eines Halteelements der ersten Transporteinrichtung. Dadurch kann diese bei gleicher Abschirmleistung besonders klein und mit geringem Materialaufwand realisiert werden.

Bei einer weiteren bevorzugten Ausführungsform verläuft wenigstens eine Wandung der Strahlungsabschirmungseinrichtung parallel zu einem Abschnitt des Transportpfads der Behältnisse. Durch diesen Verlauf kann insgesamt die Größe der Strahlungsabschirmungseinrichtung verringert werden. Insbesondere handelt es sich bei der Strahlungsabschirmungseinrichtung um eine entlang des Transportpfads der Kunststoffvorformlinge zumindest abschnittsweise torusförmige Strahlungsabschirmungseinrichtung.

Weitere Vorteile und Ausführungsformen ergeben sich aus den beigefügten Zeichnungen.

Darin zeigen:
- Fig. 1: eine schematische Aufsicht auf eine Behältnisbehandlungsvorrichtung in einer beispielhaften Ausführungsform;
- Fig. 2: eine perspektivische Ansicht der in Fig. 1 dargestellten Ausführungsform von schräg oben;
- Fig. 3: eine Aufsicht auf den Übergabebereich ohne oberen Teil der Strahlungsabschirmungseinrichtung;
- Fig. 4: eine Schnittdarstellung der Behältnisbehandlungsvorrichtung im Bereich der Behältnisübergabe;
- Fig. 5: eine Schnittdarstellung des Einlaufs in die Strahlungsabschirmungseinrichtung;
- Fig. 6: eine Schnittdarstellung des Auslaufs aus der Strahlungsabschirmungseinrichtung;
- Fig. 7: eine schematische Schrägaufsicht auf eine Behältnisbehandlungsvorrichtung mit vor- und nachgeschalteten Behältnisbehandlungseinrichtungen; und
- Fig. 8: eine weitere schematische Schrägaufsicht auf eine Behältnisbehandlungsvorrichtung mit vor- und nachgeschalteten Behältnisbehandlungseinrichtungen in einem Zustand mit geöffneter Strahlungsabschirmungseinrichtung.

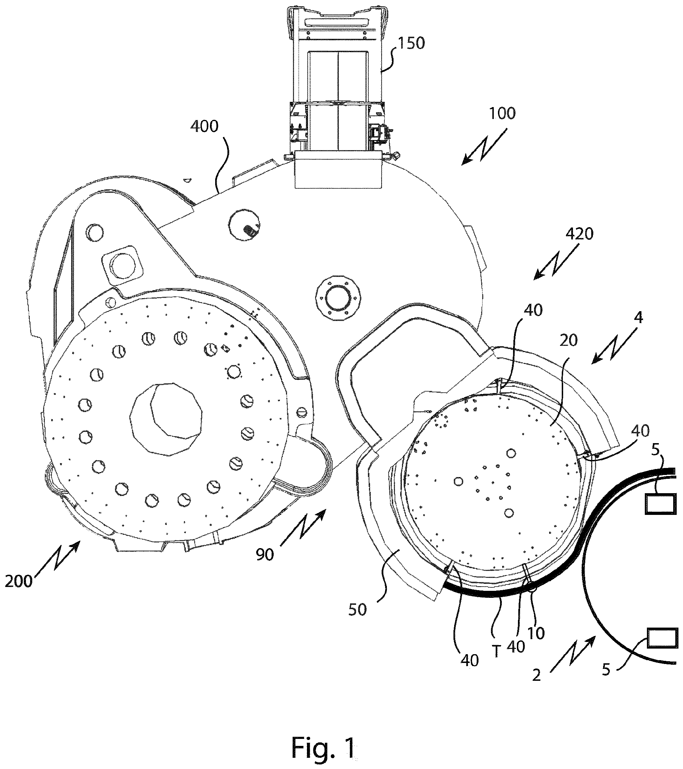

Figur 1 zeigt eine schematische Aufsicht auf eine Behältnisbehandlungsvorrichtung 1 in einer beispielhaften Ausführungsform. Die gezeigte Behältnisbehandlungsvorrichtung 1, bei der es sich im gezeigten Beispiel um eine Behältnissterilisationsvorrichtung handelt, weist drei Transporteinrichtungen 100, 200, 300 zum Transport eines Behältnisses 10 entlang eines in dieser Darstellung nicht hervorgehobenen Transportpfads T innerhalb eines Gehäuses 400 auf. Da die Transporteinrichtungen 100, 200, 300 die Behältnisse 10 im Inneren des einen Reinraum ausbildenden Gehäuses 400 führen, sind die Transporteinrichtungen 100, 200, 300 und die Behältnisse 10 in dieser Darstellung nicht oder lediglich abschnittsweise dargestellt.

Eine der Transporteinrichtungen 100, 200, 300 ist eine Behältnisaußenflächenbehandlungseinrichtung 100, von der eine Behältnisaußenflächenbeaufschlagungseinrichtung 150 beziehungsweise eine Strahlungsquelle 150 als von außen zugängliches Anbauteil am Gehäuse 400 angeordnet ist. Von einer Behältnisinnenflächenbehandlungseinrichtung 200 sind ebenfalls lediglich außerhalb des Gehäuses 400 liegende Teile sichtbar. Diese können beispielsweise Teile eines Hubmechanismus 280 sein, mittels welchem eine (hier nicht gezeigte) Behältnisinnenflächenbeaufschlagungseinrichtung 250, beispielsweise ein Strahlfinger 250 bezüglich einem Behältnis 10 relativbewegbar ist. Die Transporteinrichtungen 100 und 200 weisen jeweils eine Vielzahl von Halteeinrichtungen 140, 240 zum Halten mindestens eines Behältnisses 10 während einer Behältnisbehandlung und/oder des Transports entlang des Transportpfads T auf.

Die Transporteinrichtung 4 ist in der gezeigten Ausführungsform stromabwärts einer (lediglich schematisch dargestellten) Heizvorrichtung 2 angeordnet, die mindestens eine Heizeinrichtung 5 wie beispielsweise einen Ofen oder eine (beispielsweise Infrarot- oder Mikrowellen-) Strahlungsquelle 5 umfasst. Die Transporteinrichtung 4 weist zur Aufnahme der Behältnisse 10 eine Vielzahl von Halteelementen 40 auf, die gemeinsam auf einem rotierbaren Träger 20 angeordnet sind.

Die Transporteinrichtung 4 ist im gezeigten Ausführungsbeispiel außerhalb des Gehäuses 400 angeordnet. Lediglich die Halteelemente 40 dringen zur Übergabe der Behältnisse 10 abschnittsweise durch eine Öffnung 420 des Gehäuses 400 in das Gehäuseinnere ein.

Auch wenn die Transporteinrichtung 4 im gezeigten Ausführungsbeispiel außerhalb des Gehäuses 400 angeordnet ist, sind die Halteelemente 40 und die daran angeordneten Behältnisse 10 nicht oder lediglich teilweise erkennbar, da sie zumindest abschnittsweise in einer Strahlungsabschirmungseinrichtung 50 geführt werden. Die Strahlungsabschirmungseinrichtung 50 erstreckt sich in diesem Ausführungsbeispiel abschnittsweise entlang des Transportpfads T entlang dem die Behältnisse 10 geführt werden.

Außerdem erstreckt sich Strahlungsabschirmungseinrichtung 50 auch in den Bereich der Öffnung 420 des Gehäuses 400. Dadurch kann sie sowohl die im Bereich der Öffnung 420 des Gehäuses 400 austretende Strahlung als auch Strahlung, die sich entlang des Weges, über den die Halteelemente 40 der Transporteinrichtung 4 geführt werden, ausbreitet, effektiv abgeschirmt werden.

Als der der Behandlung in der Behältnisbehandlungsvorrichtung 1 nachgeschaltete weitere Behandlung der Behältnisse kann beispielsweise deren Umformung zu anderen Behältnissen 10, wie beispielsweise Flaschen, durch eine nicht dargestellte Umformungseinrichtung erfolgen. Ergänzend oder alternativ dazu wäre das Befüllen (und gegebenenfalls Verschlie-ßen) der durch die Behältnisbehandlungsvorrichtung 1 behandelten Behältnisse 10 denkbar.

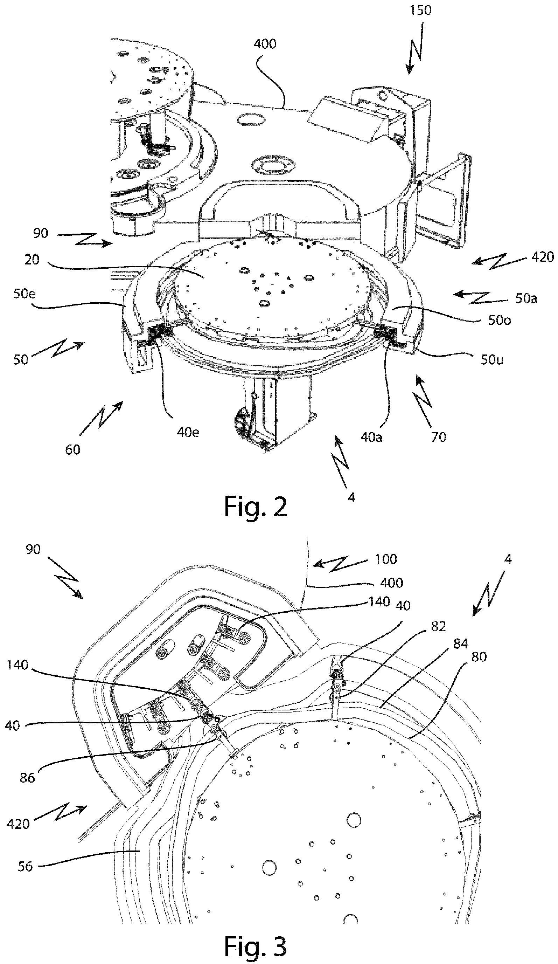

Figur 2 zeigt einen Ausschnitt der in Fig. 1 dargestellten Ausführungsform der Behältnisbehandlungsvorrichtung 1 in einer perspektivischen Ansicht von schräg oben. Im Vordergrund ist die Transporteinrichtung 4 zu sehen. Die diesbezüglich stromabwärts befindliche Heizvorrichtung 2 ist nicht dargestellt.

Die Transporteinrichtung 4 weist zur Aufnahme der Behältnisse 10 eine Vielzahl von Halteelementen 40 auf, von denen ein Halteelement 40e im Bereich des Einlaufs 60 der Strahlungsabschirmungseinrichtung 50 angeordnet ist und ein anderes Halteelement 40a im Bereich des Auslaufs 70 der Strahlungsabschirmungseinrichtung 50 angeordnet ist. Ein Behältnis 10 ist in keinem dieser beiden Halteelemente 40a und 40e angeordnet, jedoch wäre im Normalbetrieb das im Bereich des Einlaufs 60 der Strahlungsabschirmungseinrichtung 50 angeordnete Halteelement 40e mit einem Behältnis 10 bestückt.

Um ein Behältnis 10 entlang des in diesem Abschnitt von der Strahlungsabschirmungseinrichtung 50 umgebenen Transportpfads T führen zu können, ist die Strahlungsabschirmungseinrichtung 50 in dem Abschnitt 50e zwischen dem Einlauf 60 und dem Übergabebereich 90, der sich im Bereich der Öffnung 420 in dem Gehäuse 400 befindet, derart kanalartig ausgebildet, dass ein Behältnis darin geführt werden kann. Dazu weist die Strahlungsabschirmungseinrichtung 50 in dem Abschnitt 50e einen Kanal 56 auf, der sich entlang eines Abschnitts des Transportpfads T erstreckt, und in dem die Behältnisse entlang des Transportpfads T führbar sind.

Da in dem Abschnitt 50a zwischen dem Übergabebereich 90 und dem Auslauf 70 üblicherweise keine Behältnisse geführt werden, ist in diesem Bereich kein Kanal vorhanden, durch den Behältnisse geführt werden könnten. Der durch die Strahlungsabschirmungseinrichtung 50 ausgebildete Kanal weist in diesem Abschnitt 50a einen geringeren Querschnitt auf als im Abschnitt 50e. der Kanal ist aber auch im Abschnitt 50a ausreichend weit, so dass die Halteelemente 40 durchgeführt werden können und somit der Rotation des rotierbaren Trägers 20 folgen können. Die Begriffe Halteelement und Halteeinrichtung werden im Rahmen dieser Beschreibung synonym verwendet.

Weiterhin ist zu erkennen, dass die Strahlungsabschirmungseinrichtung 50 zweiteilig ausgebildet ist und einen oberen Strahlungsabschirmungseinrichtungsteil 50o und einen unteren Strahlungsabschirmungseinrichtungsteil 50u umfasst. Durch diese mehrteilige Ausgestaltung ist es möglich, die Strahlungsabschirmungseinrichtung 50 durch das Auseinanderbewegen der beiden Strahlungsabschirmungseinrichtungsteile 50o und 50u zu öffnen, beispielsweise zu Reinigungs- oder Wartungszwecke.

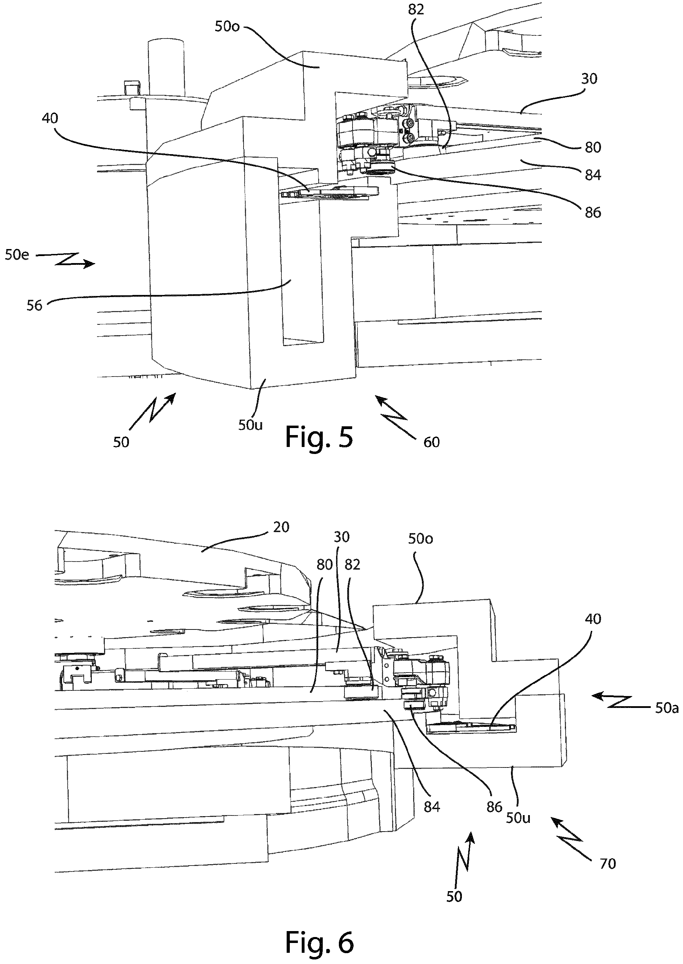

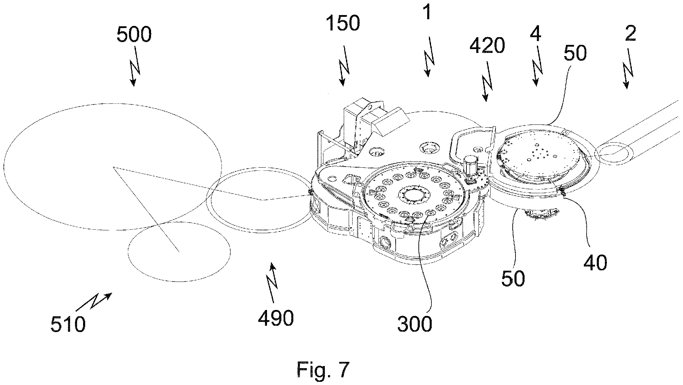

Die Ausgestaltungen des Einlaufs 60 der Strahlungsabschirmungseinrichtung 50 und des Auslaufs 70 der Strahlungsabschirmungseinrichtung 50 sind im Zusammenhang mit den Figuren 6 und 7 genauer dargestellt und beschrieben.

Figur 3 zeigt eine Aufsicht auf den Übergabebereich 90 ohne oberen Teil 50o der Strahlungsabschirmungseinrichtung 50. In einem wie in Fig. 3 dargestellten Zustand ist der obere Strahlungsabschirmungseinrichtungsteil 50o der Strahlungsabschirmungseinrichtung 50 von dem unteren Strahlungsabschirmungseinrichtungsteil 50u entfernt, so dass der innerhalb der Strahlungsabschirmungseinrichtung 50 liegende Bereich, wie beispielsweise der Kanal 56, zugänglich wird, beispielsweise für Reinigungs- oder Wartungsarbeiten.

In diesem Zustand sind auch die Halteelemente 40 zu sehen. Insbesondere ist auch der Übergabebereich 90 zu erkennen, welcher sich in einer Öffnung 420 in der Gehäusewandung erstreckt. In diesem Übergabebereich 90 werden die Behältnisse 10 von einem Halteelement 40 der ersten Transporteinrichtung 4 an eine Halteeinrichtung 140 der zweiten Transporteinrichtung 100 übergeben. In dem dargestellten geöffneten Zustand besteht ein Zugang zum Übergabebereich 90 und es ist vergleichsweise einfach möglich, die Halteelemente 40 der ersten Transporteinrichtung 4 und die Halteeinrichtungen 140 der zweiten Transporteinrichtung 100 einzustellen und derart zu justieren, dass eine sicher Übergabe der Behältnisse 10 gewährleistet wird.

Der Übergabebereich 90 stellt vorzugsweise auch eine Reinraumgrenze dar. Bereiche, die außerhalb des Gehäuses 400 liegen, werden üblicherweise nicht sterilisiert. Das Eindringen von Kontaminationen in den Reinraum kann beispielsweise durch einen Überdruck im Inneren des Gehäuses 400 verhindert werden. Durch diesen Überdruck besteht ein kontinuierlicher Gasstrom aus dem Inneren des Gehäuses 400 durch die Öffnung 420 nach außen, so dass Kontaminationen entgegen dieses Gasstroms nur äußerst beschränkt möglich sind. Die in den Reinraum im Übergabebereich 90 eingebrachten Behältnisse 10 sind zwar nicht steril, werden jedoch unmittelbar nach dem Einbringen von der zweiten Transporteinrichtung in den Wirkungsbereich der (hier nicht dargestellten Strahlungsquelle 150 gebracht und dort sterilisiert. Die Behältnisse kommen zuvor lediglich mit der zugeordneten Halteeinrichtung 140 in Kontakt, welche ebenfalls an der Strahlungsquelle 150 vorbeigeführt wird und ebenfalls sterilisiert wird. Die Gefahr einer Kontaminierung ist somit äußerst gering.

Außerdem sind in Figur 3 Steuerkurven 70, 80 erkennbar, mittels der die Halteelemente 40 gegenüber dem rotierbaren Träger 20 relativ steuerbar sind. Dazu sind Führungsrollen 82, 86 auf jeweils einer Steuerkurve 80, 84 angeordnet. Diese Führungsrollen 82, 86 rollen bei der Rotation des rotierbaren Trägers auf der jeweiligen Steuerkurve 80, 84 und bewegen einen mit der Führungsrollen 82, 86 verbundenen Mechanismus entsprechend dem radialen Abstand der Führungsrolle 82, 86 von der Rotationsachse. Dadurch lassen sich Bewegungen der Halteelemente 40 in Abhängigkeit von dem von diesem Halteelement 40 gerade bei der Rotation überstrichenen Sektor ansteuern. So ist es möglich, dass jedes Halteelement 40 an einer bestimmten Position der Kreisbahn genau eine vorgegebene Bewegung macht. Diese Bewegung kann auch eine Kombination mehrerer Bewegungen sein, welche durch verschiedene Steuerkurven 80, 84 vorgegeben sind. Beispielsweise könnte eine erste Steuerkurve 70 eine Bewegung des Halteelements 40 in radialer Richtung steuern, wohingegen eine andere Steuerkurve eine Relativbewegung des Halteelements 40 gegenüber dem rotierbaren Träger 20 entlang der Umfangsrichtung vorgibt. Ebenso könnte das Öffnen und schlie-ßen einer Halteeinrichtung 40 zum Halten eines Behältnisses oder eine Hubbewegung der Halteeinrichtung 40 entlang der Längsrichtung des Behältnisses durch eine Führungskurve gesteuert werden.

Von besonderer Bedeutung sind in diesem Zusammenhang Formen in der Steuerkurve 80, mittels welcher ein radialer Abstand einer Halteeinrichtung 40 bezüglich der Rotationsachse des rotierbaren Trägers 20 steuerbar ist. In der gezeigten Ausführungsform ist die Steuerkurve 80 so geformt, dass ein Halteelement 40 sowohl vor als auch nach dem Erreichen des Übergabebereichs 90 radial nach innen verschoben wird. Lediglich unmittelbar vor und nach der Anordnung des Halteelements 40 im Übergabebereich 90 ist das Halteelement 40 radial nach außen verschoben. Durch diese Ausgestaltung wechselt der von dem Halteelement 40 vor und nach dem Übergabebereich 90 zurückgelegte Weg mehrfach den Krümmungsradius.

Die Änderung des Krümmungsradius kann einen Wechsel von einem starken Krümmungsradius zu einem geringen Krümmungsradius (mit gleichem Vorzeichen und damit gleicher Krümmungsrichtung) und umgekehrt einschließen. Insbesondre ist jedoch bevorzugt, dass der Krümmungsradius mindestens einmal das Vorzeichen (also die Krümmungsrichtung) ändert. Der Kanal 56 folgt vorzugsweise dieser Krümmung und weist eine entsprechende Änderung seines Krümmungsradius auf.

Durch die Änderung des Krümmungsradius kann erreicht werden, dass die Strahlungsabschirmungseinrichtung 50 entlang des Verlaufs des Kanals einfallende Stählung dennoch effektiv vor dem Austreten in die Umgebung hindert, da in Bereichen mit besonders starkem Krümmungsradius eine besonders effektive Reflexion und/oder Absorption der Strahlung erfolgt. Die Strahlungsabschirmungseinrichtung 50 bildet durch den (gegebenenfalls mehrmaligen) Wechsel des Krümmungsradius und/oder der Krümmungsrichtung eine Strahlenfalle aus. Vorzugsweise wird die Strahlung in diesem Bereich mehrfach reflektiert und/oder absorbiert, so dass die Intensität der Strahlung abgeschwächt wird und/oder die Strahlung wieder in das Gehäuseinnere reflektiert wird.

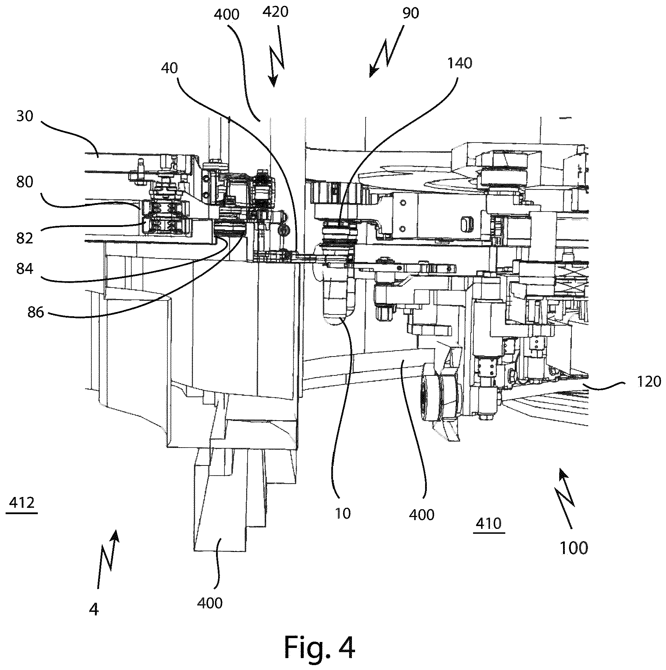

Figur 4 zeigt eine Schnittdarstellung der Behältnisbehandlungsvorrichtung 1 im Bereich der Behältnisübergabe 90.

Links ist ein Abschnitt der ersten Transporteinrichtung 4 dargestellt. So ist ein Arm 30 gezeigt, an welchem ein Halteelement 40 befestigt ist. Dieser Arm 30 kann über die auf den Steuerkurven 80 und 84 rollenden Führungsrollen 82 und 86 relativ bezüglich des rotierbaren Trägers 20 bewegt werden. So ist es beispielsweise möglich, die Halteeinrichtung 40 im Bereich der Öffnung 420 in der Gehäusewand 400 radial auswärts in Richtung einer Halteeinrichtung 140 der zweiten Transporteinrichtung 100 zu schieben.

Im Übergabebereich 90 ist ein Behältnis 10 gezeigt, welches von zwei Halteeinrichtungen 40, 140 kontaktiert wird. Dieses Behältnis 10 wird folglich gerade von der Halteeinrichtung 40 der ersten Transporteinrichtung 4 an eine Halteeinrichtung 140 der zweiten Transporteinrichtung 100 übergeben. Nachdem die Halteeinrichtung 140 der zweiten Transporteinrichtung 100 das Behältnis sicher erfasst hat, kann die Halteeinrichtung 40 der ersten Transporteinrichtung 4 das Behältnis freigeben und beispielsweise bezüglich des rotierbaren Trägers 20 radial nach innen zurückgezogen werden. Dies kann beispielsweise mittels einer der Steuerkurven 80, 84 erfolgen.

Das Behältnis kann dann an der Halteeinrichtung 140 der zweiten Transporteinrichtung 100 weiterbewegt werden, beispielsweise durch Rotation des rotierbaren Trägers 120, an welchem die Halteeinrichtung 140 angeordnet ist.

Durch eine solche Übergabe von der ersten Transporteinrichtung 4 an die zweiten Transporteinrichtung 100 ist es möglich, ein Behältnis durch die Öffnung 420 in der Gehäusewand 400 von einem unsterilen Außenbereich 412 in den Reinraum 410 einzubringen.

Zumindest eine Halteeinrichtung 140 der Behältnisaußenflächenbehandlungseinrichtung 100 kann zumindest zeitweise in den Bereich einer Öffnung 420 der Gehäusewandung 400 gebracht werden. Dadurch wird ermöglicht, dass dieses Halteelement 140 aus einem Raum 412 außerhalb des Gehäuses 400 von einer dort stromaufwärts bezüglich des Transportpfads angeordneten Transporteinrichtung 4 ein Behältnis aufnehmen kann. Durch diese Ausgestaltung kann auf eine zusätzliche Transporteinrichtung innerhalb des Gehäuses verzichtet werden, die das Behältnis von außerhalb aufnimmt und an die Behältnisaußenflächenbehandlungseinrichtung 100 weiterleitet.

Figur 5 zeigt eine Schnittdarstellung des Einlaufs 60 in die Strahlungsabschirmungseinrichtung 50. An den Einlauf 60 schließt sich entlang des Transportpfades T der Abschnitt 50e der Strahlungsabschirmungseinrichtung 50 zwischen dem Einlauf 60 und dem Übergabebereich 90 an.in diesem Bereich ist die Strahlungsabschirmungseinrichtung 50 mindestens zweiteilig ausgebildet und umfasst einen oberen Strahlungsabschirmungseinrichtungsteil 50o und einen unteren Strahlungsabschirmungseinrichtungsteil 50u. Gemeinsam bilden diese Strahlungsabschirmungseinrichtungsteile 50u und 50o eine Ummantelung des Transportpfads, welche diesen in einer senkrechten Querschnittsrichtung so umschließt, dass Strahlung nicht direkt austreten kann. Falls Strahlung überhaupt austreten könnte, wäre dies lediglich nach mehrmaliger Reflexion an den inneren Oberflächen der Strahlungsabschirmungseinrichtung 50 und dem damit verbundenen Energieverlust möglich.

Zwischen den beiden Strahlungsabschirmungseinrichtungsteilen 50u und 50o ist bevorzugt auf der radial innenliegenden Seite ein Spalt durch den Teile der Halteeinrichtung 40 hindurchgeführt werden können. So kann die Halteeinrichtung 40 über einen Arm 30 mit dem rotierbaren Träger verbunden sein und dennoch das (nicht gezeigte) Behältnis 10 im Inneren des durch die Strahlungsabschirmungseinrichtung 50 gebildeten Kanals 56 geführt werden.

In Figur 5 sind weiterhin die Steuerkurven 80 und 84 gut sichtbar, welche durch Wechselwirkung mit den Führungsrollen 82 beziehungsweise 86 eine Steuerung des Halteelements 40 bewirken können.

Figur 6 zeigt eine Schnittdarstellung des Auslaufs 70 aus der Strahlungsabschirmungseinrichtung 50. Der Auslauf 70 schließt entlang des Transportpfades T den Abschnitt 50a der Strahlungsabschirmungseinrichtung 50 ab, der zwischen dem Übergabebereich 90 und dem Auslauf 70 angeordnet ist. in diesem Abschnitt ist die Strahlungsabschirmungseinrichtung 50 vorzugsweise ebenfalls mindestens zweiteilig ausgebildet und umfasst einen oberen Strahlungsabschirmungseinrichtungsteil 50o und einen unteren Strahlungsabschirmungseinrichtungsteil 50u. In diesem Abschnitt 50a werden jedoch üblicherweise keine Behältnisse durch die Strahlungsabschirmungseinrichtung 50 geführt, daher ist die Ausbildung eines vergleichsweise großen Kanals nicht notwendig. Vielmehr kann der Bereich zwischen der oberen Strahlungsabschirmungseinrichtungsteil 50o und der unteren Strahlungsabschirmungseinrichtungsteil 50u so schmal sein, dass lediglich die Halteeinrichtung 40 zwischen diesen beiden Strahlungsabschirmungseinrichtungsteilen 50o und 50u hindurchgeführt werden kann.

Durch den geringen Raum zwischen den beiden Strahlungsabschirmungseinrichtungsteilen 50u und 50o ist der Austritt von Strahlung besonders gut gehemmt. Falls Strahlung überhaupt austreten könnte, wäre dies lediglich nach mehrmaliger Reflexion an den inneren Oberflächen der Strahlungsabschirmungseinrichtung 50 und dem damit verbundenen Energieverlust möglich. Die Anzahl der Reflexionen und damit auch der Energieverlust ist aufgrund der im Vergleich zu dem Abschnitt 50e wesentlich geringeren freie Strecke zwischen den Innenwandungen der Strahlungsabschirmungseinrichtung 50 deutlich erhöht. Der Austritt energiereicher Strahlung kann so effektiv verhindert werden.

Wie auch in Figur 5 sind auch in Figur 6 die Steuerkurven 80 und 84 gut sichtbar, welche durch Wechselwirkung mit den Führungsrollen 82 beziehungsweise 86 eine Steuerung des Halteelements 40 bewirken können.

Figur 7 zeigt eine schematische Schrägaufsicht auf eine Behältnisbehandlungsvorrichtung 1 mit vor- und nachgeschalteten Behältnisbehandlungseinrichtungen 2, 500. Bei den dargestellten Behältnisbehandlungseinrichtungen 2, 500 handelt es sich im gezeigten Bespiel einerseits um eine Heizvorrichtung 2, welche stromaufwärts der ersten Transporteinrichtung 4 angeordnet ist und andererseits um eine Behältnisumformungseinrichtung 500. In der Behältnisumformungseinrichtung 500 werden beispielsweise Vorformlinge zu Flaschen umgeformt, vorzugsweise (form-) geblasen. Der Behältnisumformungseinrichtung 500 ist ein Einlaufstern 490 vorgeschaltet und ein Auslaufstern 510 nachgeschaltet.

Die Behältnisbehandlungsvorrichtung 1 ist im gezeigten Beispiel eine Sterilistationseinrichtung, in der die Behältnisse von der Strahlungsquelle 150 mit sterilisierender Strahlung beaufschlagt werden. Ebenso ist in der Behältnisbehandlungsvorrichtung 1 eine Behältnisinnensterilisation im Bereich der Transporteinrichtung 300 vorgesehen. Zumindest einige der dazu notwendigen Einrichtungen sind jedoch in Figur 7 nicht dargestellt.

Die Strahlungsabschirmungseinrichtung 50 ist in Figur 7 in einem geschlossenen Zustand dargestellt. Der obere Strahlungsabschirmungseinrichtungsteil 50o liegt somit auf dem unteren Strahlungsabschirmungseinrichtungsteil 50u auf, so dass eine bündig abgeschlossene Barriere für aus dem Übergabebereich austretende Strahlung besteht.

In dem oberen Strahlungsabschirmungseinrichtungsteil 50o ist in der in Figur 7 dargestellten Ausgestaltung ein Öffnung 58 vorgesehen, durch die ein Fluid in den Übergabebereich 90 geleitet werden kann. Die Öffnung kann daher auch als Fluidanschluss bezeichnet werden. Durch das Zuführen eines (vorzugsweise sterilen) Fluids in den Übergabebereich kann dort lokal ein Überdruck erzeugt werden, der bewirkt, dass das Fluid, vorzugsweise ein Gas, durch die Öffnung 420 aus dem Gehäuse 400 ausströmt. Dadurch kann das Einströmen von Verunreinigungen entgegen dieses Fluidstroms effektiv verhindert werden. Das Einbringen der Behältnisse in das Gehäuseinnere wird dadurch jedoch nicht maßgeblich behindert.

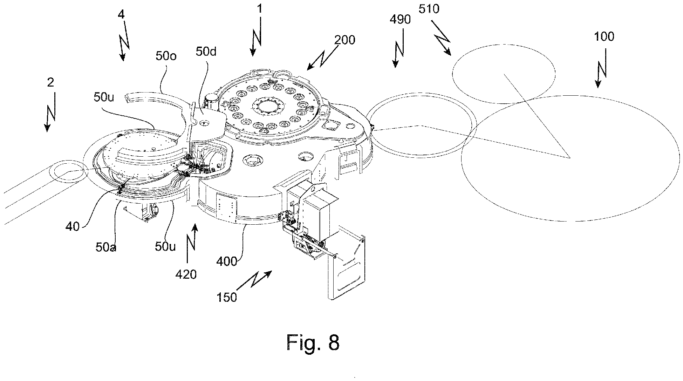

Figur 8 zeigt eine weitere schematische Schrägaufsicht auf eine Behältnisbehandlungsvorrichtung mit vor- und nachgeschalteten Behältnisbehandlungseinrichtungen in einem Zustand mit geöffneter Strahlungsabschirmungseinrichtung 50.

Die in Figur 8 dargestellten der Behältnisbehandlungsvorrichtung 1 vor- und nachgeschalteten Behältnisbehandlungseinrichtungen 2, 500 entsprechen den in Figur 7 dargestellten. So handelt es sich um eine stromaufwärts der ersten Transporteinrichtung 4 angeordnete Heizvorrichtung 2 und eine stromabwärts der Behältnisbehandlungsvorrichtung 1 angeordnete Behältnisumformungseinrichtung 500. Der Behältnisumformungseinrichtung 500 ist auch hier ein Einlaufstern 490 vorgeschaltet und ein Auslaufstern 510 nachgeschaltet.

Bei der Behältnisbehandlungsvorrichtung 1 handelt es sich wiederum um eine Sterilistationseinrichtung, mit einer Behältnisaußensterilisation durch die Strahlungsquelle 150 und einer Behältnisinnenstrerilistaion im Bereich der Transporteinrichtung 300.

Die Strahlungsabschirmungseinrichtung 50 ist in Figur 8 in einem geöffneten Zustand dargestellt, bei dem der obere Strahlungsabschirmungseinrichtungsteil 50o nach oben von dem unteren Strahlungsabschirmungseinrichtungsteil 50u entfernt wurde. Dadurch ist der zwischen diesen beiden Strahlungsabschirmungseinrichtungsteilen 50o und 50u gebildete Raum zugänglich, beispielsweise für Reinigungs- oder Wartungsarbeiten.

Insbesondere ist in diesem Zustand jedoch der Übergabebereich zugänglich. Dadurch kann vergleichsweise einfach dieser Bereich gewartet werden und/oder die Übergabe der Behältnisse korrekt eingestellt werden. Der Übergabebereich hat sich als besonders kritisch erwiesen, weshalb eine besonders genaue Einstellung der Übergabe notwendig ist, insbesondere also der Abstimmung der Bewegung der Haltelemente 40 der ersten Transporteinrichtung 4 und der Haltelemente 140 der zweiten Transporteinrichtung 100, gegebenenfalls unter Berücksichtigung der Geometrie der zu übergebenden Behältnisse.

In einer besonders bevorzugten Ausführungsform ist das obere Strahlungsabschirmungseinrichtungsteil 50o mindestens zweiteilig ausgebildet. Insbesondere ist bevorzugt, dass ein Teil 50d des oberen Strahlungsabschirmungseinrichtungsteil 50o, welches vorzugsweise zumindest abschnittsweise eine Abdeckung der Öffnung 420 in dem Gehäuse 400 ist, separat bewegbar ist. Dadurch wird ermöglicht, dass der besonders kritische Bereich im Übergabebereich separat zugänglich gemacht werden kann oder nicht. So kann es beispielsweise vorteilhaft sein, diesen Bereich abgedeckt zu lassen, wenn beispielsweise eine Wartung aufgrund eines im Abschnitt 50a der Strahlungsabschirmungseinrichtung 50 zwischen dem Übergabebereich 90 und dem Auslauf 70 aufgrund eines dort angeordneten, nicht korrekt übergebenen Behältnisses notwendig ist. Andererseits kann eine separate Öffnung des Teils 50d des oberen Strahlungsabschirmungseinrichtungsteil 50o dann sinnvoll sein, wenn beispielsweise eine Wartung oder Justage im Bereich der Übergabe 90 notwendig, eine Kontamination des Kanals zwischen oberem und unterem Strahlungsabschirmungseinrichtungsteil 50o und 50u vermieden werden soll.

Die Anmelderin behält sich vor, sämtliche in den Anmeldungsunterlagen offenbarten Merkmale als erfindungswesentlich zu beanspruchen, sofern sie einzeln oder in Kombination gegenüber dem Stand der Technik neu sind. Es wird weiterhin darauf hingewiesen, dass in den einzelnen Figuren auch Merkmale beschrieben wurden, welche für sich genommen vorteilhaft sein können. Der Fachmann erkennt unmittelbar, dass ein bestimmtes in einer Figur beschriebenes Merkmal auch ohne die Übernahme weiterer Merkmale aus dieser Figur vorteilhaft sein kann. Ferner erkennt der Fachmann, dass sich auch Vorteile durch eine Kombination mehrerer in einzelnen oder in unterschiedlichen Figuren gezeigter Merkmale ergeben können.

### Bezugszeichenliste

- 1: Behältnisbehandlungsvorrichtung
- 2: Heizvorrichtung
- 4: Erste Transporteinrichtung, Transportstern (außerhalb des Gehäuses)
- 5: Heizeinrichtung
- 6: Antriebseinrichtung der Transporteinrichtung bzw. des Transportsterns 4
- 10: Behältnis, Vorformling
- 11: Mündung des Kunststoffvorformlings
- 14: Tragring
- 20: rotierbarer Träger
- 30: Arm
- 40: Halteelement
- 50: Strahlungsabschirmungseinrichtung
- 50o: oberer Strahlungsabschirmungseinrichtungsteil
- 50u: unterer Strahlungsabschirmungseinrichtungsteil
- 50e: Abschnitt der Strahlungsabschirmungseinrichtung zwischen dem Einlauf und dem Übergabebereich
- 50a: Abschnitt der Strahlungsabschirmungseinrichtung zwischen dem Übergabebereich und dem Auslauf
- 58: Öffnung, Fluidanschluss
- 60: Einlauf
- 70: Auslauf
- 80: Steuerkurve
- 82: Führungsrolle
- 84: Steuerkurve
- 86: Führungsrolle
- 90: Übergabebereich, Bereich der Behältnisübergabe
- 100: Behältnisaußenflächenbehandlungseinrichtung, zweite Transporteinrichtung
- 105: Hub- und Dreheinrichtung
- 110: Verschiebeeinrichtung, Verschiebemechanismus
- 120: rotierbarer Träger
- 132: Rotor der Hub- und Dreheinrichtung
- 140: Halteelement, Halteeinrichtung, Dorn
- 150: Behältnisaußenflächenbeaufschlagungseinrichtung, Strahlungsquelle
- 200: Behältnisinnenflächenbehandlungseinrichtung, Transporteinrichtung
- 300: Transporteinrichtung, Transportstern (innerhalb des Gehäuses)
- 400: Gehäuse
- 410: Raum innerhalb des Gehäuses, Gehäuseinneres, Reinraum
- 412: Raum außerhalb des Gehäuses, Umgebung
- 420: Fenster, Öffnung (zum Einschleusen in das Gehäuseinnere)
- 490: Einlaufstern
- 500: Blasformeinrichtung
- 510: Auslaufstern

- T: Transportpfad

## Patentansprüche

1. Behältnisbehandlungsvorrichtung (1) zum Transportieren von Kunststoffbehältnissen (10) und insbesondere Kunststoffvorformlingen entlang eines vorgegebenen Transportpfads (T), wobei die Behältnisbehandlungsvorrichtung (1) mehrere Transporteinrichtungen (4, 100, 200, 300) aufweist, die jeweils mindestens ein Halteelement (140, 240) zum Halten eines Kunststoffbehältnisses (10) und Führen des Kunststoffbehältnisses (10) auf einem Abschnitt des Transportpfads (T) aufweisen, wobei eine zweite Transporteinrichtung (100) entlang des Transportpfads (T) unmittelbar auf eine erste Transporteinrichtung (4) folgt und Behältnisse von einer Halteeinrichtung (40) der ersten Transporteinrichtung (4) an eine Halteeinrichtung (140) der zweiten Transporteinrichtung (100) übergebbar sind,
**dadurch gekennzeichnet, dass**
der Transportpfad, (T) entlang dem die Behältnisse durch die erste Transporteinrichtung (4) führbar sind, zumindest abschnittsweise außerhalb eines Reinraums (410) angeordnet ist und der Transportpfad (T) entlang dem die Behältnisse (10) durch die erste Transporteinrichtung (4) führbar sind, zumindest abschnittsweise von einer Strahlungsabschirmungseinrichtung (50) umgeben ist.

2. Behältnisbehandlungsvorrichtung (1) nach Anspruch 1,
**dadurch gekennzeichnet, dass**
der Transportpfad (T) entlang dem die Behältnisse (10) durch die zweite Transporteinrichtung (100) führbar sind, zumindest abschnittsweise innerhalb eines Reinraums (410) angeordnet ist.

3. Behältnisbehandlungsvorrichtung (1) nach einem der vorangegangenen Ansprüche,
**dadurch gekennzeichnet, dass**
es sich bei der ersten Transporteinrichtung (4) und/oder bei der zweiten Transporteinrichtung (100) um einen Teilungsverzugsstern handelt.

4. Behältnisbehandlungsvorrichtung (1) nach einem der vorangegangenen Ansprüche,
**dadurch gekennzeichnet, dass**
der Transportpfad (T), entlang dem die Behältnisse durch die zweite Transporteinrichtung (100) führbar sind, zumindest abschnittsweise in einem Einflussbereich einer Behältnisaußenflächenbehandlungseinrichtung (150) zum Behandeln von Außenflächen der Kunststoffbehältnisse (10), insbesondere eine Behältnisaußenflächensterilisationseinrichtung und/oder einer Behältnisinnenflächenbehandlungseinrichtung (200) zum Behandeln von Innenflächen der Kunststoffbehältnisse (10), insbesondere eine Behältnisinnenflächensterilisationseinrichtung, verläuft.

5. Behältnisbehandlungsvorrichtung (1) nach einem der vorangegangenen Ansprüche,
**dadurch gekennzeichnet, dass**
sich die Strahlungsabschirmungseinrichtung (50) ausgehend von einem Übergabepunkt (90) oder Übergabebereich(90), an welchem Behältnisse von einer Halteeinrichtung (40) der ersten Transporteinrichtung (4) an eine Halteeinrichtung (140) der zweiten Transporteinrichtung (100) übergebbar sind, abschnittsweise entlang des Transportpfads (T) erstreckt auf welchem die Behältnisse (10) durch die erste Transporteinrichtung (4) führbar sind, und vorzugsweise auch entlang einer Richtung, in welcher die Halteeinrichtung (40) der ersten Transporteinrichtung (4) nach der Übergabe eines Behältnisses (10) an eine Halteeinrichtung der zweiten Transporteinrichtung (100) bewegbar ist, insbesondere bevorzugt entlang eines Kreisbogens, auf welchem die Halteeinrichtung (40) der ersten Transporteinrichtung (4) führbar ist.

6. Behältnisbehandlungsvorrichtung (1) nach einem der vorangegangenen Ansprüche,
**dadurch gekennzeichnet, dass**
die Strahlungsabschirmungseinrichtung (50) einen Pfad, auf dem eine Halteeinrichtung (40) der ersten Transporteinrichtung (4) bewegbar ist, zumindest abschnittsweise umschließt und/oder die Strahlungsabschirmungseinrichtung (50) einen Querschnitt aufweist, welcher im Wesentlichen die Form des Buchstaben Omega (Ω) hat.

7. Behältnisbehandlungsvorrichtung (1) nach einem der vorangegangenen Ansprüche,
**dadurch gekennzeichnet, dass**
die Strahlungsabschirmungseinrichtung (50) mindestens zweiteilig ausgebildet ist, wobei sich vorzugsweise die mindestens zwei Strahlungsabschirmungseinrichtungsteile (50o, 50u) voneinander trennbar sind, vorzugsweise durch einen Antrieb voneinander beabstandbar sind.

8. Behältnisbehandlungsvorrichtung (1) nach einem der vorangegangenen Ansprüche,
**dadurch gekennzeichnet, dass**
die Strahlungsabschirmungseinrichtung (50) in einem ersten Strahlungsabschirmungsbereich (50e), welcher entlang des Transportpfads (T) stromaufwärts eines Übergabepunkts (90) oder Übergabebereichs (90), an welchem Behältnisse von einer Halteeinrichtung (40) der ersten Transporteinrichtung (4) an eine Halteeinrichtung (140) der zweiten Transporteinrichtung (100) übergebbar sind, angeordnet ist, in zumindest einer senkrecht zum Transportpfad (T) angeordneten Richtung einen größeren Querschnitt aufweist als in einem zweiten Strahlungsabschirmungsbereich (50a), in welchem die Halteeinrichtung (40) der ersten Transporteinrichtung (4) nach der Übergabe eines Behältnisses (10) an eine Halteeinrichtung (140) der zweiten Transporteinrichtung (100) bewegbar ist.

9. Behältnisbehandlungsvorrichtung (1) nach einem der vorangegangenen Ansprüche,
**dadurch gekennzeichnet, dass**
die Strahlungsabschirmungseinrichtung (50) zumindest abschnittsweise nicht eine Reinraumgrenze ist, wobei vorzugsweise die Strahlungsabschirmungseinrichtung (50) zumindest in einem Bereich keine Reinraumgrenze bildet, in welchem sich die Strahlungsabschirmungseinrichtung (50) entlang eines Pfads erstreckt, auf dem eine Halteeinrichtung (40) der ersten Transporteinrichtung (4) bewegbar ist.

10. Behältnisbehandlungsvorrichtung (1) nach einem der vorangegangenen Ansprüche,
**dadurch gekennzeichnet, dass**
ein Weg auf dem ein Halteelement der ersten Transporteinrichtung führbar ist, mindestens einmal, vorzugsweise mehrfach den Krümmungsradius ändert, wobei diese Änderung des Krümmungsradius einen Wechsel von einem starken Krümmungsradius zu einem geringen Krümmungsradius und umgekehrt einschließt und/oder mindestens einen einmaligen Wechsel des Vorzeichens des Krümmungsradius einschließt.

11. Verfahren zum Transportieren von Kunststoffbehältnissen (10) und insbesondere Kunststoffvorformlingen (10) entlang eines vorgegebenen Transportpfads (T), wobei die Kunststoffbehältnisse (10) durch mehrere Transporteinrichtungen (4, 100, 200, 300) transportiert werden, wobei jeweils mindestens ein Halteelement (140, 240) ein Kunststoffbehältnis (10) hält und dieses Kunststoffbehältnis (10) auf einem Abschnitt des Transportpfads (T) führt, wobei ein Kunststoffbehältnis von einer Halteeinrichtung (40) einer ersten Transporteinrichtung (4) an eine Halteeinrichtung (140) einer zweiten Transporteinrichtung (100) übergeben wird,
**dadurch gekennzeichnet, dass**
die Behältnisse (10) durch die erste Transporteinrichtung (4) auf einem Transportpfad (T) geführt werden, der zumindest abschnittsweise außerhalb eines Reinraums (410) angeordnet ist und die Behältnisse (10) durch die erste Transporteinrichtung (4) auf einem Transportpfad (T) geführt werden, der zumindest abschnittsweise von einer Strahlungsabschirmungseinrichtung (50) umgeben ist.
